# EUROPEAN PATENT APPLICATION

(11) **EP 4 417 074 A2**
(43) Date of publication of application: **21.08.2024**
(21) Application number: 24157961.4
(22) Date of filing: 15.02.2024
(51) Int. Cl.: A24F 40/40, A24F 40/485

(54) **VAPING APPARATUS**

(30) Priority: 17.02.2023 US 202363485668 P
(71) Applicant: Kind Distribution LLC, Austin, TX 78702 (US)
(72) Inventor: Atalla, Elie, Harrington Park, 07640 (US); Bissen, Patrick, Austin, 78741 (US)
(74) Representative: Alpspitz IP

(57) **Abstract**

Vaping apparatus includes a housing defining a heating chamber and an intake port to admit ambient air into the heating chamber. The heating chamber includes a bowl, an inner cylindrical wall beneath the bowl defining a central heating cavity, an intermediate cylindrical wall around the inner cylindrical wall that defines therewith an intermediate heating cavity, and an outer cylindrical wall around the intermediate cylindrical wall that defines therewith an outer heating cavity. The various heating cavities are in fluid communication with each other and the bowl. A heating element is positioned within a heating cavity. A removable adapter, which includes a void between its upstream and downstream ends, may be attached to the housing. An elastomeric sealing element positioned at the upstream end of the shell forms an airtight vapor path between the bowl and the void. The elastomeric sealing element may be isolated from the vapor path.

## Description

### BACKGROUND

The instant disclosure relates generally to vaping devices. In particular, the instant disclosure relates to vaping devices with improved heating ability and maintainability.

People currently use vaping devices (also referred to as "vaporizers"), which use electricity or fire to heat up loose leaf tobacco, other botanicals, or vaping liquid contained within a heating chamber for inhalation of the resulting vapor through a mouthpiece. The heating chamber generally includes a bowl to contain the vaping material, and a heating cavity, including a heating element, beneath the bowl to heat air that will be passed across the vaping material for inhalation.

Ambient air typically enters extant vaping devices through an air intake, including one or more holes, in the bottom of the vaping device when the user inhales through the device mouthpiece. The air is typically drawn directly upwards from the intake, heats as it is drawn across the heating element, into the bowl where it forms a vapor of the vaping material, through the mouthpiece and into the user's mouth.

To create a sealed vapor path between the mouthpiece and the main body, a sealing material, such as an elastomeric material, is often provided between the mouthpiece and the main body. A portion of this material, however, is typically within the vapor path, where it is susceptible to more rapid degradation necessitating replacement.

### BRIEF SUMMARY

Disclosed herein is a vaping apparatus including: a housing defining a heating chamber, the heating chamber including: a bowl; an inner cylindrical wall positioned beneath the bowl, the inner cylindrical wall defining a central heating cavity, wherein the central heating cavity is in fluid communication with the bowl; an intermediate cylindrical wall positioned around the inner cylindrical wall, the intermediate cylindrical wall and the inner cylindrical wall defining therebetween an intermediate heating cavity, wherein the intermediate heating cavity is in fluid communication with the central heating cavity; and an outer cylindrical wall positioned around the intermediate cylindrical wall, the outer cylindrical wall and the intermediate cylindrical wall defining therebetween an outer heating cavity, wherein the outer heating cavity is in fluid communication with the intermediate heating cavity; a heating element positioned within at least one of the central heating cavity, the intermediate heating cavity, and the outer heating cavity; and an intake port extending through the housing to permit ambient air to enter the outer heating cavity.

In embodiments of the disclosure, the heating element is positioned within the central heating cavity.

The intermediate cylindrical wall can include one or more through holes to establish the fluid communication between the outer heating cavity and the intermediate heating cavity. According to certain aspects of the disclosure, the through holes may be positioned on an end of the intermediate cylindrical wall opposite the intake port.

The vaping apparatus can include a diffuser between the central heating cavity and the bowl. The diffuser can include a plurality of apertures arranged to shift airflow from the central heating cavity towards a perimeter of the bowl.

An adapter may be removably and releasably attached to the housing adjacent the heating chamber. The adapter can include: a shell, having an upstream end and a downstream end, wherein the shell defines a first aperture at the upstream end, a second aperture at the downstream end, and a void extending between the first aperture and the second aperture, wherein the void is in fluid communication with the bowl through the first aperture; and an elastomeric sealing element positioned at the upstream end of the shell to form an airtight vapor path between the bowl and the void, and wherein the elastomeric sealing element is isolated from the vapor path.

For example, the upstream end of the shell can include a cylindrical insert portion, which in turn includes an external circumferential groove, and the elastomeric sealing element may be positioned within the external circumferential groove. The insert portion may extend into the bowl when the adapter is attached to the housing. The insert portion may also include a screen to prevent uptake of vaping material.

A vaping mouthpiece may be secured within the void of the adapter. In embodiments of the disclosure, the vaping mouthpiece may be removably secured within the void. Thus, it is contemplated that the vaping mouthpiece may be selected from a plurality of vaping mouthpieces, with the void configured to removably (and therefore interchangeably) receive each of the plurality of vaping mouthpieces through the second aperture. A second elastomeric sealing element may be positioned within the void proximate the upstream end to form an airtight seal between the adapter and one of the plurality of vaping mouthpieces.

An optionally removable power supply may be disposed within the housing and operable to energize the heating element.

Also disclosed herein is a vaping apparatus including: a housing defining a heating chamber, the heating chamber including: a bowl; a heating cavity positioned beneath the bowl and in fluid communication with the bowl; a heating element positioned within the heating cavity; an intake port extending through the housing to permit ambient air to enter the heating cavity; and an adapter removably and releasably attached to the housing adjacent the heating chamber. The adapter includes: a shell, having an upstream end and a downstream end, wherein the shell defines a first aperture at the upstream end, a second aperture at the downstream end, and a void extending between the first aperture and the second aperture, wherein the void is in fluid communication with the bowl through the first aperture; and an elastomeric sealing element positioned at the upstream end of the shell to form an airtight vapor path between the bowl and the void, and wherein the elastomeric sealing element is isolated from the vapor path.

In embodiments of the disclosure, the heating cavity includes: a central heating cavity in fluid communication with the bowl, wherein the central heating cavity is defined by an inner cylindrical wall positioned beneath the bowl; an intermediate heating cavity in fluid communication with the central heating cavity, wherein the intermediate heating cavity is defined by the inner cylindrical wall and an intermediate cylindrical wall positioned around the inner cylindrical wall; and an outer heating cavity in fluid communication with the intermediate heating cavity, wherein the outer heating cavity is defined by the intermediate cylindrical wall and an outer cylindrical wall positioned around the intermediate cylindrical wall.

The upstream end of the shell can include a cylindrical insert portion, and wherein the elastomeric sealing element can be positioned around an external surface of the cylindrical insert portion. For example, in embodiments of the disclosure, the external surface of the cylindrical insert portion includes a circumferential groove into the external surface, and the elastomeric sealing element is positioned within the circumferential groove. A screen to prevent uptake of vaping material may also be secured to the cylindrical insert portion.

The instant disclosure also relates to an adapter for a vaping apparatus. The adapter includes: a shell, having an upstream end and a downstream end, wherein the shell defines a first aperture at the upstream end, a second aperture at the downstream end, and a void extending between the first aperture and the second aperture, the upstream end of the shell including a cylindrical insert portion having an exterior surface; and an elastomeric sealing element positioned around the exterior surface of the cylindrical insert portion to form an airtight vapor path between the void and a bowl of the vaping apparatus. In certain embodiments of the disclosure, the elastomeric sealing element is isolated from the vapor path.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a front, left, and top perspective view of a vaping apparatus according to embodiments disclosed herein.
Figure 2 is a front, right, and top perspective view of the vaping apparatus shown in Figure 1.
Figure 3 is a front cutaway view of the vaping apparatus shown in Figure 1.
Figure 4 is a close-up of region 4 in Figure 3.
Figure 5 is a left side cutaway view of the vaping apparatus shown in Figure 1.
Figure 6 is a cutaway view of an adapter for a vaping apparatus according to embodiments disclosed herein.
Figure 7 is an exploded view of an insert portion for an adapter for a vaping apparatus according to embodiments disclosed herein.

While multiple embodiments are disclosed, still other embodiments of the present disclosure will become apparent to those skilled in the art from the following detailed description, which shows and describes illustrative embodiments. Accordingly, the drawings and detailed description are to be regarded as illustrative in nature and not restrictive.

### DETAILED DESCRIPTION

The instant disclosure relates to vaping apparatus. For purposes of illustration, aspects of the disclosure will be described with reference to a vaping device, such as the Planet of the Vapes^{®} TWO portable vaporizer (Kind Distribution LLC; Austin, TX). Those of ordinary skill in the art will understand, however, how to apply the teachings herein to good advantage in other contexts and/or with respect to other devices having different overall configurations.

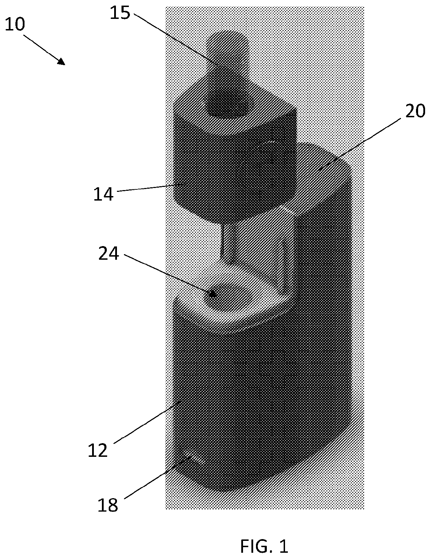

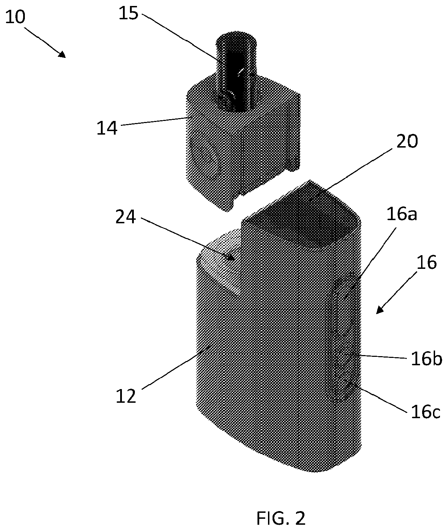

Figures 1 and 2 depict a vaping apparatus 10, generally including a housing 12 and an adapter 14. Adapter 14 accommodates a mouthpiece 15 (or, as discussed below, a selected one of a grouping of interchangeable mouthpieces).

Those of ordinary skill in the art will be familiar with various features of vaping apparatus 10, such as controls 16 (*e.g.* a power button 16a and temperature adjustment buttons 16b, 16c), a power supply 17 (*e.g.,* an internal rechargeable battery) and charging port 18, a display 20, associated electronics, and the like. Accordingly, further aspects of vaping apparatus 10 will be described herein only to the extent necessary for an understanding of the instant disclosure.

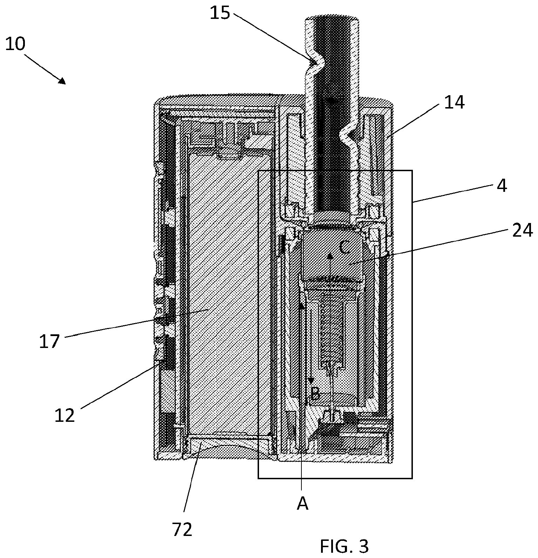

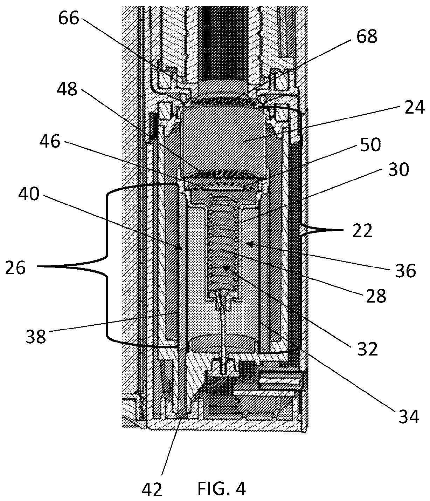

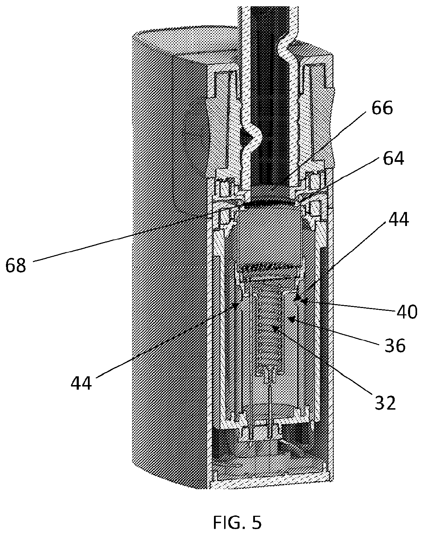

Figures 3 and 5 are front and side cutaway views of vaping apparatus 10 revealing the interior of housing 12 and adapter 14. Figure 4 is a close-up of region 4 in Figure 3. As shown in Figures 3-5, housing 12 defines a heating chamber 22, which generally includes a bowl 24 and a heating cavity 26 beneath and in fluid communication with bowl 24. A heating element 28, such as an electrically-conductive coil, is disposed within heating cavity 26; in some aspects of the disclosure, heating element 28 is centrally-disposed within heating cavity 26. As will be described in further detail below, when a user inhales through mouthpiece 15, ambient air is drawn into heating cavity 26, heated by heating element 28, and drawn into bowl 24 to produce an inhalable vapor of desired vaping material.

According to certain embodiments disclosed herein, heating cavity 26 includes a plurality of separate compartments that collectively define an advantageously convoluted airflow pathway. For instance, an inner cylindrical wall 30 can define a central heating cavity 32; an intermediate cylindrical wall 34 positioned around inner cylindrical wall 30 can define therewith an annular intermediate heating cavity 36; and an outer cylindrical wall 38 positioned around intermediate cylindrical wall 34 can define therewith an annular outer heating cavity 40.

It should be understood that the phrase "cylindrical" is used broadly herein and is not intended to be limited, for example, to right circular cylindrical structures. Rather, the term "cylindrical" is intended to be inclusive of cylindrical structures that have non-circular (*e.g*., elliptical) bases and/or structures that vary in transverse cross-sectional configuration along their height dimension (*e.g*., structures that exhibit tapering).

An intake port 42 incorporating one or more airholes extends through housing 12 to allow ambient air to enter heating cavity 26 (*e.g.,* outer heating cavity 40) when a user inhales through mouthpiece 15. Air will first travel upwards through outer heating cavity 40 (arrow "A") until it reaches through holes 44 in intermediate cylindrical wall 34, which are desirably opposite intake port 42 and allow passage into intermediate heating cavity 36. Once in intermediate heating cavity 36, the air will reverse directions and travel downwards (arrow "B"). Once the air reaches the open bottom end of inner cylindrical wall 30, it will reverse direction yet again, passing into central heating cavity 32 and traveling upwards (arrow "C"), eventually passing into bowl 24 through a diffuser 46 and a first screen 48.

Diffuser 46 includes a plurality of apertures 50 that direct airflow from central heating cavity 36 towards the perimeter of bowl 24. First screen 48 supports a vaping material (*e.g.,* dry herbs) to be heated within bowl 24.

Advantageously, the convoluted airflow pathway described above allows more time for air to heat before entering bowl 24 when compared to extant vaping apparatus, because ambient air will make, in effect, three passes through the heating zone prior to entering bowl 24. Thus, the vaping material within bowl 24 can be exposed to higher-temperature air during a vaping session without having to increase the power to heating element 28. That is, for a given power supplied to heating element 28, the convoluted airflow pathway according to the instant disclosure will yield higher-temperature air at bowl 24 than a conventional vaping apparatus, which generally allows only a single pass through the heating zone prior to entering bowl 24.

Adapter 14 will be further described with reference to Figures 5-7. Adapter 14 is typically releasably and removably secured to housing 12, such as by snap-fit connectors (*e.g*., friction-fit tabs), magnetic connectors, or the like. By removing adapter 14, a user can access bowl 24 (*e.g.,* to add, remove, or change vaping material, or for maintenance).

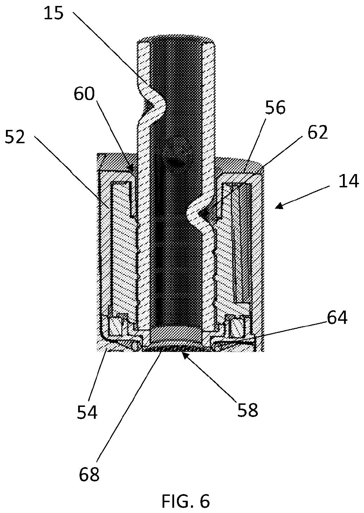

As shown in Figure 6, adapter 14 includes a shell 52 having an upstream end 54 (*e.g.,* closest to bowl 24) and a downstream end 56 (*e.g.,* furthest from bowl 24). Shell 52 defines a first aperture 58 at upstream end 54, a second aperture 60 and downstream end 56, and a void 62 extending between first and second apertures 58, 60. Thus, when adapter 14 is secured to housing 12, void 62 is in fluid communication with bowl 24 through first aperture 58 as shown to good advantage in Figures 3-5.

Adapter 14 further includes an elastomeric sealing element 64, such as an o-ring, positioned at upstream end 54. Suitable materials for elastomeric sealing element 64 include various heat resistant and fire-retardant materials, such as silicone rubber. When adapter 14 is secured to housing 12, elastomeric sealing element 64 is compressed between adapter 14 and housing 12 to form an airtight seal therebetween, thus defining an airtight vapor path between bowl 24 and void 62.

As used herein, the term "airtight" is not limited to a completely airtight arrangement. Rather, the term "airtight" is intended to encompass some amount of leakage (*e.g.,* between adapter 14 and housing 12), provided the extent of the leakage does not impair usage of vaping apparatus 10.

The term "vapor path," however, refers only to the intended airflow pathway during normal usage of vaping apparatus 10, upwards from bowl 24 into void 62 (more particularly, into mouthpiece 15 within void 62). Thus, the term "vapor path" excludes any leakage pathway that may exist between adapter 14 and housing 12 if the seal therebetween is not completely airtight.

According to aspects of the disclosure, elastomeric sealing element 64 is isolated from (*e.g*., outside of) the vapor path between bowl 24 and void 62. This reduces the presence of silicone in the vapor path, which may be desirable for several reasons. For instance, curing compound byproducts that remain on silicone may produce an unpleasant odor and/or a bad taste in the user's mouth if placed in the vapor path. As another example, silicone in the vapor path is susceptible to break down over time and repeated heating, which could degrade the use of vaping apparatus 10. By eliminating silicone in the vapor path, and replacing it with inert materials (*e.g.,* glass, stainless steel, high-temperature plastic, and the like), these disadvantages can be minimized or eliminated.

Accordingly, in some embodiments of the disclosure, upstream end 54 of adapter 14 includes a cylindrical insert portion 66, and elastomeric sealing element 64 is positioned around an external surface of cylindrical insert portion 66, such as within an external circumferential groove 68 into the external surface of cylindrical insert portion 66. Insert portion 66 may protrude from upstream end 54 of adapter 14 such that, when adapter 14 is secured to housing 12, insert portion 66 may extend a short distance into bowl 24. In general, however, when adapter 14 is secured to housing 12, elastomeric sealing element 64 will be compressed between insert portion 66 and housing 12 to form the airtight seal therebetween.

Insert portion 66 may be integrally formed with the remainder of adapter 14. Alternatively, insert portion 66 may be separately formed from the remainder of adapter 14 and either permanently (*e.g.,* through the use of an adhesive) or removably (*e.g.,* through the use of a suitable fastening mechanism, such as a snap-fit or magnetic connection) secured thereto. Suitable materials for insert portion 66 include, without limitation, various high-temperature plastics (*e.g*., polyether ether ketone (PEEK), polyether ketone ketone (PEKK), polyetherimide (PEI), and the like).

As those of ordinary skill in the art will recognize, upstream end 54 may also include a second screen 68 to permit the flow of vapor from bowl 24 into adapter 14 (*e.g.,* void 62) and out through mouthpiece 15 (that is, along the vapor path), but to prevent uptake of vaping material from bowl 24 when the user inhales. Second screen 68 may be secured within insert portion 66 and may, in certain embodiments of the disclosure, be integrally formed therewith.

Although it is contemplated that mouthpiece 15 may be fixedly secured to adapter 14 within void 62, embodiments of the instant disclosure relate to mouthpieces 15 that are removably and interchangeably received within void 62. Thus, a user can readily change between various vaping mouthpieces, such as straight pipes, bent pipes, bubblers, and other mouthpiece configurations, without removing adapter 14 from housing 12, which would undesirably release heat to ambient.

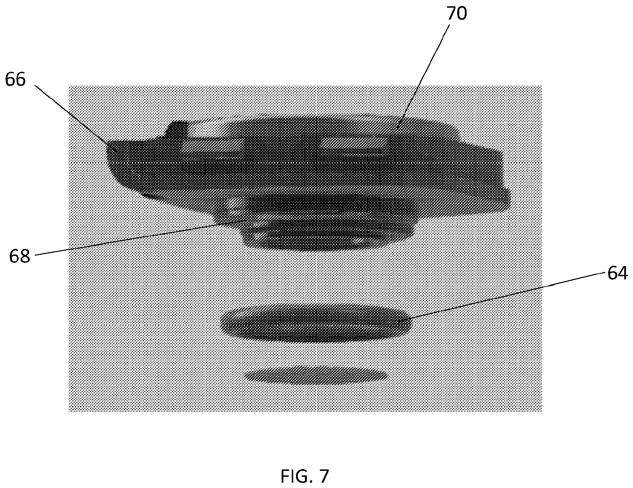

To create an airtight seal between a selected mouthpiece 15 and adapter 14, and thus preserve the airtight vapor path described above, insert portion 66 can include a second elastomeric sealing element 70 as shown in Figure 7. A mouthpiece 15 inserted into void 62 can either abut against second elastomeric sealing element 70 or be received within an opening of an annular elastomeric sealing element 70.

In use, a user fills bowl 24 with vaping material of choice and then secures adapter 14 to housing 12. This compresses elastomeric sealing element 64 between adapter 14 (*e.g*., insert portion 66) and housing 12, thus forming an airtight vapor path from bowl 24 into void 62.

If necessary, the user can then choose a desired vaping mouthpiece 15 and insert one end thereof into void 62 through second aperture 60. When fully inserted, the end of mouthpiece 15 will seal against second elastomeric sealing element 70 (*e.g.,* by abutting against or passing into the center of second elastomeric sealing element 70), thereby reinforcing the airtight vapor path described above.

The user can then turn vaping apparatus 10 on, which will supply power to, and begin heating, heating element 28 and, in turn, air within heating cavity 26. By inhaling through mouthpiece 15, the user draws ambient air into housing 12, along the convoluted airflow pathway described above (*e.g*., upwards through outer heating cavity 40, downwards through intermediate heating cavity 36, and upwards through central heating cavity 32) where it is heated, thus drawing heated air upwards through diffuser 46 and screen 48 into bowl 24, and finally along the vapor path from bowl 24 into void 62/mouthpiece 15, to inhale a vapor of the vaping material in bowl 24 through mouthpiece 15.

Although several embodiments of this invention have been described above with a certain degree of particularity, those skilled in the art could make numerous alterations to the disclosed embodiments without departing from the spirit or scope of this invention.

For example, the power supply 17 may be user-accessible, such as through door 72, to allow the user to remove and replace a depleted power supply.

All directional references (*e.g*., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g*., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the spirit of the invention as defined in the appended claims.

## Claims

1. An adapter (14) for a vaping apparatus (10), the adapter (14) comprising:
a shell (52), having an upstream end (54) and a downstream end (56), wherein the shell (52) defines a first aperture (58) at the upstream end (54), a second aperture (60) at the downstream end (56), and a void (62) extending between the first aperture (58) and the second aperture (60),
the upstream end (54) of the shell (52) including a cylindrical insert portion (66) having an exterior surface; and
an elastomeric sealing element (64) positioned around the exterior surface of the cylindrical insert portion (66) to form an airtight vapor path between the void (62) and a bowl (24) of the vaping apparatus (10).

2. The adapter (14) according to claim 1, wherein the elastomeric sealing element (64) is isolated from the vapor path.

3. A vaping apparatus (10) comprising:
a housing (12) defining a heating chamber (22), the heating chamber comprising:
a bowl (24);
a heating cavity (26) positioned beneath the bowl (24) and in fluid communication with the bowl (24);
a heating element (28) positioned within the heating cavity (26);
an intake port (42) extending through the housing (12) to permit ambient air to enter the heating cavity (26).

4. The vaping apparatus (10) according to claim 3, wherein the heating cavity (26) comprises:
a central heating cavity (32) in fluid communication with the bowl (24), wherein the central heating cavity (32) is defined by an inner cylindrical wall (30) positioned beneath the bowl (24);
an intermediate heating cavity (36) in fluid communication with the central heating cavity (32), wherein the intermediate heating cavity (36) is defined by the inner cylindrical wall (30) and an intermediate cylindrical wall (34) positioned around the inner cylindrical wall (30); and
an outer heating cavity (40) in fluid communication with the intermediate heating cavity (36), wherein the outer heating cavity (40) is defined by the intermediate cylindrical wall (34) and an outer cylindrical wall (38) positioned around the intermediate cylindrical wall (34).

5. The vaping apparatus (10) according to claim 4, wherein the heating element (28) is positioned within the central heating cavity (32).

6. The vaping apparatus (10) according to claim 4 or 5, wherein the intermediate cylindrical wall (34) includes one or more through holes (44) to establish the fluid communication between the outer heating cavity (40) and the intermediate heating cavity (36).

7. The vaping apparatus according to claim 6, wherein the one or more through holes (44) are positioned on an end of the intermediate cylindrical wall (34) opposite the intake port (42).

8. The vaping apparatus (10) according to any one of claims 4 to 7, further comprising a diffuser (46) between the central heating cavity (32) and the bowl (24), wherein the diffuser (46) comprises a plurality of apertures (50) arranged to shift airflow from the central heating cavity (32) towards a perimeter of the bowl (24).

9. The vaping apparatus (10) according to any one of claims 3 to 8, further comprising an adapter (14) according to claim 1 or 2, wherein the adapter (14) is removably and releasably attached to the housing (12) adjacent the heating chamber (22), and wherein the void (62) is in fluid communication with the bowl (24) through the first aperture (58).

10. The vaping apparatus (10) according to claim 9, wherein, with the adapter (14) attached to the housing (12), the insert portion (66) extends into the bowl (24).

11. The vaping apparatus (10) according to claim 9 or 10, wherein the upstream end (54) of the shell (52) includes a cylindrical insert portion (66), and wherein the elastomeric sealing element (64) is positioned around an external surface of the cylindrical insert portion (66).

12. The vaping apparatus (10) according to claim 11, wherein the external surface of the cylindrical insert portion (66) includes a circumferential groove (68) into the external surface, and wherein the elastomeric sealing element (64) is positioned within the circumferential groove (68).

13. The vaping apparatus (10) according to any one of claims 9 to 12, wherein the adapter (14) further comprises a screen secured to the cylindrical insert portion (66).

14. The vaping apparatus (10) according to claim 9, further comprising a vaping mouthpiece (15) secured within the void (62),
wherein preferably the vaping mouthpiece (15) is removably secured within the void (62) and/or
wherein preferably the vaping mouthpiece (15) is selected from a plurality of vaping mouthpieces (15), and the void (62) is configured to removably receive each of the plurality of vaping mouthpieces (15) through the second aperture (60).

15. The vaping apparatus (10) according to claim 14, further comprising a second elastomeric sealing element (64) positioned within the void (62) proximate the upstream end (54) to form an airtight seal between the adapter(14) and one of the plurality of vaping mouthpieces (15) and/or further comprising a power supply (17) removably disposed within the housing (12) and operable to energize the heating element (28).
